(19) **Europäisches Patentamt**
**European Patent Office**
**Office européen des brevets**

(11) **EP 2 168 474 B1**

(12) **FASCICULE DE BREVET EUROPEEN**

(45) Date de publication et mention
de la délivrance du brevet:
**02.07.2014 Bulletin 2014/27**

(51) Int Cl.:
*A61B 3/107* *(2006.01)*   *A61B 3/11* *(2006.01)*

(21) Numéro de dépôt: **09290707.0**

(22) Date de dépôt: **17.09.2009**

(54) **Dispositif et procédé de mesure automatique des rayons cornéens des deux yeux d'un individu**

Vorrichtung und Verfahren zur automatischen Messung der Hornhautkrümmung beider Augen einer Person

Device and Method for automatically measuring the corneal radius of an individual's two eyes

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **25.09.2008 FR 0805271**

(43) Date de publication de la demande:
**31.03.2010 Bulletin 2010/13**

(73) Titulaire: **ESSILOR INTERNATIONAL (Compagnie Générale d'Optique)**
**94220 Charenton le Pont (FR)**

(72) Inventeur: **Baranton, Konogan**
**94220 Charenton Le Pont (FR)**

(74) Mandataire: **Chauvin, Vincent et al**
**Coralis**
**14/16, rue Ballu**
**75009 Paris (FR)**

(56) Documents cités:
**EP-A- 1 444 945       WO-A-02/11612**
**WO-A1-2004/010860     US-A- 5 777 718**
**US-A1- 2003 142 271**

**Description**

DOMAINE TECHNIQUE AUQUEL SE RAPPORTE L'INVENTION

La présente invention concerne de manière générale l'examen ophtalmologique et en particulier la kératométrie. Elle vise spécifiquement un procédé de kératométrie ainsi qu'un kératomètre automatique pour la mesure d'au moins une grandeur kératométrique relative à la géométrie de la cornée de chacun des deux yeux d'un individu.

ARRIÈRE-PLAN TECHNOLOGIQUE

La kératométrie automatique est une mesure objective de grandeurs, dites kératométriques, relatives à la géométrie de la cornée d'un oeil d'un individu. Cette mesure sert notamment à la préparation et l'assistance aux interventions chirurgicales de l'oeil et à l'adaptation des lentilles de contact à la courbure de l'oeil à corriger. Il s'agit le plus souvent de mesurer les rayons de courbure de la face antérieure de la cornée dans ses deux méridiens principaux.

L'opérateur, ophtalmologue ou opticien, utilise à cet effet un instrument appelé kératomètre.

Un instrument de ce type est décrit dans le document US4597648. Cet instrument permet la kératométrie d'un oeil d'un individu subissant ou devant subir une opération de chirurgie de l'oeil en question. Il s'agit d'un appareil monoculaire : la mesure est effectuée individuellement pour un oeil unique. L'instrument comporte :

- un boîtier,
- une pluralité de sources lumineuses de configuration géométrique connue montées dans le boîtier de manière à éclairer l'oeil à mesurer de l'individu,
- un système optique d'imagerie monté dans le boîtier et agencé pour fournir une image du reflet cornéen des sources lumineuses sur l'oeil,
- au moins un capteur photoélectrique monté sur le boîtier pour capturer cette image et adapté à convertir cette image en un signal qui en est représentatif,
- un calculateur adapté à traiter ledit signal pour calculer les grandeurs kératométriques recherchées de l'oeil.

Ce type d'instrument donne satisfaction en ce qui concerne la mesure d'un oeil dans un référentiel absolu, mais s'avère d'utilisation fastidieuse tant pour l'opérateur que l'individu subissant la kératométrie. En particulier, pour mesurer les deux yeux de l'individu, l'opérateur doit effectuer séquentiellement deux mesures distinctes, l'une pour l'oeil gauche et l'autre pour l'oeil droit. L'opérateur doit, pour chacun des deux yeux, positionner avec précaution la tête de l'individu dans le référentiel de l'instrument afin que les deux yeux soient mesurés dans un même référentiel. Cette contrainte se traduit immanquablement par une complexité opératoire et/ou instrumentale.

On connaît par ailleurs du document WO 02/11612 un dispositif de mesure de caractéristiques d'un oeil, faisant simultanément office de kératomètre et de WMD (Wavefront Measuring Device) pour mesurer les erreurs de réfraction de l'oeil (ou aberrations).

Dans ce document, le dispositif de mesure est toutefois dépourvu de moyens de calcul de l'écart inter-pupillaire ou des demi-écarts pupillaires des deux yeux de l'individu.

On connaît également du document US 5 777 718 un réfractomètre automatique qui comporte deux chemins optiques pour afficher simultanément deux images cibles en regard des deux yeux du patient, et qui est agencé pour mesurer les valeurs de réfraction des deux yeux du patient de telle manière que les effets d'ajustement des yeux lorsqu'ils travaillent seuls n'influent pas sur les mesures.

Dans ce document, le réfractomètre présente une fonction de pupillomètre pour mesurer l'écart inter-pupillaire des deux yeux de l'individu. Il comporte à cet effet des moyens d'alignement des deux chemins optiques dans l'axe des deux yeux du patient et un potentiomètre dont la résistance varie en fonction de l'écart entre les deux chemins optiques.

Ce dispositif ne présente toutefois pas une fonction de kératomètre. Sa fonction de pupillomètre est par ailleurs mise en oeuvre à l'aide d'un système électro-mécanique onéreux.

OBJET DE L'INVENTION

Un but de la présente invention est de proposer un kératomètre et un procédé de kératométrie permettant de fournir, avec efficience et économie de moyens, des mesures pertinentes pour la conception personnalisée d'un équipement de correction visuelle.

A cet effet, on propose selon l'invention un procédé de kératométrie automatique pour la mesure d'au moins une grandeur kératométrique relative à la géométrie de la cornée de chacun des deux yeux d'un individu, tel que défini dans la revendication 1.

Plus particulièrement selon l'invention, les reflets cornéens des deux yeux sont capturés, tour à tour ou simultanément, sans déplacement relatif du visage de l'individu par rapport au capteur.

On propose aussi selon l'invention un kératomètre automatique tel que défini dans la revendication 7.

Les deux yeux de l'individu sont préférentiellement mesurés simultanément. Par simultanément on entend que les captures des images des reflets cornéens des deux yeux sont réalisées à un même instant ou à des instants rapprochés situés dans un intervalle de temps maximum d'un dixième de seconde.

L'homme du métier n'avait jusqu'à ce jour en aucun cas envisagé la kératométrie globale des deux yeux sans déplacement de l'instrument de mesure par rapport à la tête de l'individu sujet de la kératométrie. Il considérait au contraire nécessaire à la précision de la mesure que soient effectués tour à tour deux relevés monoculaires, sur chaque oeil séparément, au moyen d'une instrumentation optique et d'un protocole de prise de mesure dédiés à un seul oeil (mesure monoculaire) et ainsi réputée précise. L'état de la technique n'identifiait par ailleurs pas d'intérêt à effectuer la kératométrie pour les deux yeux globalement et l'homme du métier, l'eut-il envisagé, ne pouvait alors y percevoir qu'une source de complication optique, facteur d'augmentation des coûts.

C'est à l'encontre de ce préjugé que les travaux de la demanderesse l'ont amenée à identifier un inconvénient spécifique à la prise de mesure individuelle de chaque oeil, notamment lorsque la kératométrie est effectuée à des fins de conception personnalisée d'un équipement de correction visuelle tel que des lentilles de contact, un implant intra-oculaire ou des lentilles de lunettes.

Une telle conception s'appuie sur un ensemble de mesures physiologiques et son efficacité dépend, non seulement de la précision de ces mesures, mais également, au moins pour certaines de ces mesures, du contexte dans lequel elles sont effectuées (qui doit s'approcher autant que possible des conditions du porté de l'équipement visuel) et de la cohérence des différentes mesures entre elles. La demanderesse considère en particulier comme important, au moins dans certaines circonstances, que les deux yeux soient mesurés dans des conditions identiques ou analogues, faute de quoi la correction fournie au porteur de l'équipement visuel sera déséquilibrée et le confort visuel du porteur risque alors de se voir fortement dégradé.

S'agissant en particulier de la kératométrie, la demanderesse a identifié que le confort visuel du porteur pouvait être amélioré en effectuant la mesure des deux yeux dans une configuration de prise de mesure commune, sans déplacement de la tête, c'est-à-dire dans un même contexte de sollicitation du système visuel de l'individu.

La mesure sans déplacement des deux yeux permet en outre, avantageusement mais non nécessairement, une prise de mesure à la fois commode, rapide et efficace dans des conditions moins contraignantes tant pour l'opérateur que pour l'individu sujet de la kératométrie. On s'affranchit de plus, au moins en partie, des risques d'erreur de mesure qui, dans le mode de prise de mesure monoculaire de l'état de la technique, pouvaient résulter d'un écart de positionnement de la tête de l'individu entre la prise de mesure de l'oeil droit et celle de l'oeil gauche. On évite ainsi la mise en oeuvre de complexités opératoires ou instrumentales visant à pallier ce risque de défaut de positionnement.

La pertinence de la mesure et, partant, le confort visuel du porteur peuvent encore être améliorés en effectuant la mesure des deux yeux simultanément, c'est-à-dire dans un même contexte de sollicitation du système visuel de l'individu.

Avantageusement, le système optique d'imagerie comporte au moins l'un des éléments suivants :

- une lentille ayant au moins un diamètre horizontal d'au moins dix centimètres et dont l'axe optique, éventuellement renvoyé ou dévié, aboutit au capteur photoélectrique,
- deux parties de lentilles ou deux doublets équivalant à une lentille unique dont l'axe optique, éventuellement renvoyé ou dévié, aboutit au capteur photoélectrique.

D'autres caractéristiques avantageuses et non limitatives du procédé selon l'invention sont exposées dans les revendications 2 à 6.

D'autres caractéristiques avantageuses et non limitatives du kératomètre selon l'invention sont par ailleurs exposées dans les revendications 8 à 15.

## DESCRIPTION DÉTAILLÉE D'UN EXEMPLE DE RÉALISATION

La description qui va suivre, en regard des dessins annexés, donnée à titre d'exemple non limitatif, fera bien comprendre en quoi consiste l'invention et comment elle peut être réalisée.

Sur les dessins annexés :

- la figure 1 est une vue schématique d'un kératomètre selon un premier mode de réalisation de l'invention ;
- la figure 2 est une vue en coupe du kératomètre de la figure 1, suivant le plan II-II ;
- la figure 3 est une vue en coupe du kératomètre de la figure 1, suivant le plan III-III ;
- la figure 4 est une vue en coupe du kératomètre de la figure 1, suivant le plan IV-IV ;
- la figure 5 est une vue d'ensemble du kératomètre et de son support branché à un ordinateur ;

- la figure 6 est une vue de face de la source lumineuse du kératomètre de la figure 1 ;
- la figure 7 est une vue analogue à la figure 1, illustrant un second mode de réalisation de l'invention ;
- la figure 8 est une vue schématique des chemins empruntés par deux rayons lumineux dans le kératomètre de la figure 1;
- la figure 9 est une vue schématique des chemins empruntés par quatre rayons lumineux dans un kératomètre selon un troisième mode de réalisation de l'invention ; et
- la figure 10 est une vue schématique d'une variante de réalisation du kératomètre de la figure 1.

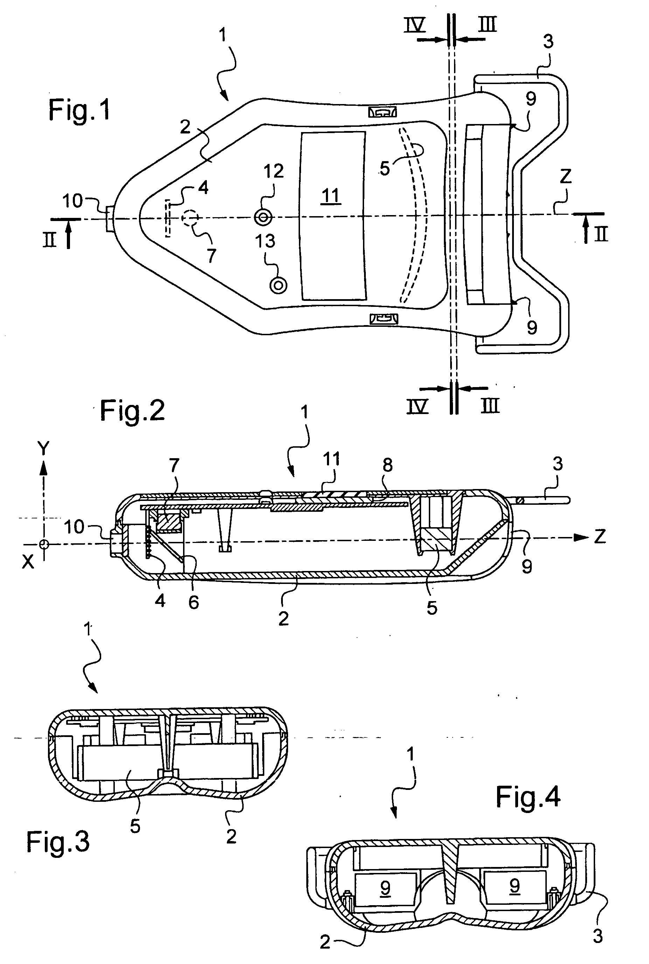

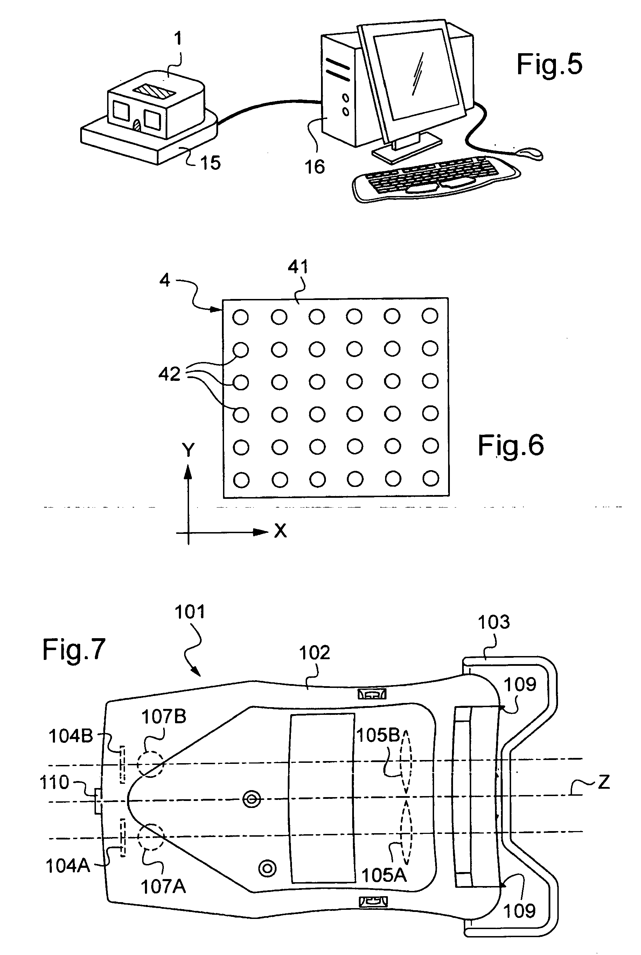

Sur les figures 1 et 7, on a représenté deux modes de réalisation d'un kératomètre automatique 1 ; 101 conforme à l'invention. Ce kératomètre automatique 1 ; 101 permet d'acquérir des grandeurs kératométriques, c'est-à-dire des grandeurs géométriques relatives à la forme et à la taille de la cornée de chacun des deux yeux d'un individu.

Il comporte à cet effet un boîtier 2 ; 102 creux et globalement parallélépipédique, allongé suivant un axe longitudinal Z.

Ce boîtier 2 ; 102 présente une première extrémité, située à droite sur les figures 1 et 7, qui est percée de deux fenêtres de mesure 9 ; 109 rectangulaires (également visibles sur les figures 2 et 4). Cette première extrémité du boîtier 2 ; 102 est en outre équipée d'une partie d'appui 3 ; 103 en forme de monture de lunettes située en regard des deux fenêtres de mesure 9 ; 109. Cette partie d'appui 3 ; 103 permet de poser de manière stable le boîtier 2 ; 102 sur le visage de l'individu, de telle manière que ses deux yeux soient situés en vis-à-vis des deux fenêtres de mesure 9 ; 109.

Le boîtier 2 ; 102 présente une seconde extrémité, située à l'opposé de la première, qui est percée d'une fenêtre d'observation 10 ; 110 disposée en vis-à-vis des deux fenêtres de mesure 9 ; 109 et au travers de laquelle un observateur peut vérifier que le boîtier 2 ; 102 est correctement positionné par rapport au visage de l'individu.

Comme le montre plus particulièrement les figures 1, 2 et 7, le boîtier 2 ; 102 loge intérieurement :

- au moins une source lumineuse 4 ; 104A, 104B de configuration géométrique connue, montée(s) dans le boîtier 2 de manière à éclairer simultanément les deux yeux de l'individu,
- un système optique d'imagerie 5 ; 105A, 105B agencé pour fournir, tour à tour ou simultanément et sans déplacement relatif du visage de l'individu par rapport au boîtier 2 ; 102, une ou deux image(s) plane(s) des reflets cornéens générés par la ou les source(s) lumineuse(s) 4 ; 104A, 104B sur les deux yeux de l'individu,
- au moins un capteur photoélectrique 7 ; 107A, 107B agencé pour capturer cette ou ces image(s) et adapté à convertir cette ou ces image(s) en un signal représentatif,
- un calculateur 8 adapté à traiter ce signal pour calculer une grandeur kératométrique de chaque oeil.

Dans un premier mode de réalisation de l'invention représenté sur les figures 1 à 4, le kératomètre automatique 1 est adapté à réaliser simultanément des mesures sur les deux yeux du porteur, au moyen d'un unique capteur photoélectrique 7 et d'une unique image sur laquelle apparaissent les reflets cornéens des deux yeux de l'individu.

Système optique d'imagerie

Le système optique d'imagerie 5 est à cet effet adapté à générer une image unitaire sur laquelle apparaissent l'un et l'autre des reflets cornéens des deux yeux de l'individu. On dit alors du kératomètre automatique 1 qu'il comporte une seule voie optique.

Tel que représenté sur les figures 1 à 3, le système optique d'imagerie 5 comporte pour cela une unique lentille de collimation 5 dont l'une des faces optiques se prolonge d'un côté à l'autre du boîtier 2, en regard des deux fenêtres de mesure 9. Cette lentille de collimation 5 présente à cet effet un bord de forme rectangulaire qui s'étend en longueur sur au moins dix centimètres, ici sur 12 centimètres. Elle est positionnée dans le boîtier 2 de manière que son axe optique se confonde avec l'axe longitudinal Z du boîtier 2 et que son plan focal image se situe à faible distance de la fenêtre d'observation 10, du côté intérieur du boîtier 2. Elle présente ici une distance focale égale à 100 millimètres.

Selon une variante non représentée de l'invention, le système optique d'imagerie pourrait comporter deux dispositifs optiques distincts équivalant à la lentille de collimation précitée, ces deux dispositifs optiques étant chacun situés en regard de l'une des fenêtres de mesure 9 du boîtier 2.

Ces dispositifs optiques pourraient par exemple être formés par deux découpes de la lentille de collimation présentant chacune la forme de l'une des fenêtres de mesure 9. Ces deux découpes seraient alors respectivement positionnées en regard des deux fenêtres de mesure 9, de telle manière que leurs axes optiques se confondent avec l'axe longitudinal Z du boîtier 2.

Ces dispositifs optiques pourraient également être formés par deux doublets comportant chacun une lentille convexe de petit diamètre adjointe à un prisme optique. Ces deux doublets seraient alors également respectivement positionnés en regard des deux fenêtres de mesure 9, de telle manière que leurs axes optiques se confondent avec l'axe longitudinal Z du boîtier 2.

Quoi qu'il en soit, ces deux dispositifs optiques distincts auraient alors la même fonction de collimation et les mêmes effets que la lentille de collimation 5 représentée sur les figures 1 à 3.

Source lumineuse

La source lumineuse 4 est centrée sur l'axe optique de la lentille de collimation 5 et est positionnée au voisinage du plan focal image de cette lentille, si bien qu'elle simule une source de lumière située à l'infini. Cette source lumineuse 4 stimule donc la vision de loin de l'individu et elle éclaire à la fois l'un et l'autre des deux yeux de cet individu. De cette manière, lorsque le boîtier 2 et correctement installé sur le visage de l'individu, celui-ci fixe un point de fixation situé à l'infini.

La source lumineuse 4 est avantageusement adaptée à se réfléchir sur la cornée de chaque oeil de l'individu en au moins deux zones distinctes et non jointives.

Telle que représentée sur les figures 2 et 6, elle est ici formée par une matrice de diodes électroluminescentes 42 rapportées sur une plaque-support 41 et pilotées en état allumé ou éteint par le calculateur 8. Ici, ces diodes électroluminescentes 42 sont réparties sur six lignes et six colonnes, et sont écartées deux à deux de 20 millimètres. Chacune de ces 36 diodes électroluminescentes est alors adaptée à se réfléchir sur la cornée de chaque oeil de l'individu en une zone isolée des zones illuminées par les 35 autres diodes électroluminescentes.

Grâce à la taille de la matrice de diodes et à la distance focale de la lentille de collimation 5, les reflets des 36 diodes apparaissant sur chaque cornée seront répartis dans un disque de 5 millimètres de diamètre centré autour de la pupille de l'oeil de l'individu.

Ainsi, grâce à ces diodes, le système optique d'imagerie 5 peut générer une image unitaire sur laquelle apparaissent 72 reflets correspondant aux reflets des 36 diodes sur les deux yeux de l'individu. Chaque ensemble de 36 reflets forme alors l'un des reflets cornéens.

La plaque-support 41 présente quant à elle une forme carrée d'environ 120 millimètres de côté. Elle est disposée dans le boîtier 2 en regard de la fenêtre d'observation 10, de telle manière que les diodes électroluminescentes 42 éclairent en direction des deux fenêtres de mesure 9. Elle est par ailleurs réalisée en plastique transparent ou en verre, de manière que l'opérateur puisse observer les yeux du porteur au travers de la fenêtre d'observation 10, de cette plaque-support 41 et des deux fenêtres de mesure 9.

Selon une variante de l'invention non représentée, la source lumineuse pourra être formée par une lampe superposée à un masque.

La lampe pourra par exemple comporter un ou plusieurs néons positionnés derrière un diffuseur de lumière permettant d'homogénéiser l'intensité lumineuse émise par la lampe.

Le masque pourra quant à lui être formé par une carte électronique accolée à une plaque transparente pourvue d'une matrice de points opaques ou par une plaque opaque pourvue d'une matrice de points transparents, aptes à se réfléchir sur les cornées de chaque oeil de l'individu. La carte électronique sera pourvue d'une ouverture centrale afin de permettre la visée par l'opérateur au travers de la fenêtre d'observation 10. Le masque pourra en variante être formé par un écran à cristaux liquides (LCD) transparent piloté par le calculateur 8 pour afficher des motifs, comme par exemple une trame de points noirs sur fond transparent ou une trame de lignes verticales ou horizontales sur fond transparent, ou tout autre motif de géométrie connue.

Quoi qu'il en soit, cette superposition de lampe et de masque présente les mêmes effets que la matrice de diodes électroluminescentes 42 représentée sur les figures 2 et 6.

Capteur photoélectrique

Le capteur photoélectrique est ici constitué d'une caméra numérique 7 agencée pour capturer l'image des deux reflets cornéens formée par le système optique d'imagerie 5, et prévue pour transformer cette image en un signal électrique représentatif.

Cette caméra 7 est préférentiellement positionnée au voisinage du plan focal image de la lentille de collimation 5, ce qui permet de constituer un système télécentrique permettant d'obtenir une image de taille invariable quel que soit l'écart, suivant l'axe Z, entre le boîtier 2 et le visage de l'individu.

A cet effet, on pourra prévoir que la plaque-support 41 soit percée d'une ouverture centrale au travers de laquelle serait inséré l'objectif de la caméra, de manière que cette dernière soit positionnée sur l'axe optique de la lentille de collimation 5, dans son plan focal image.

Toutefois, ici, il est prévu de dévier l'axe optique de la lentille de collimation 5 par une plaque semi-réfléchissante 6, de manière à dédoubler son plan focal image.

Comme le montre plus particulièrement la figure 2, cette plaque semi-réfléchissante 6 est positionnée entre la lentille de collimation 5 et la plaque-support 41 de la source lumineuse 4, et est inclinée à 45 degrés par rapport à l'axe longitudinal Z du boîtier 2.

Grâce à cette plaque semi-réfléchissante 6, la caméra peut être positionnée à distance de la source lumineuse 4, suivant un axe perpendiculaire à l'axe longitudinal Z du boîtier 2. L'agencement des différents organes du kératomètre 1 dans le boîtier 2 est ainsi simplifié.

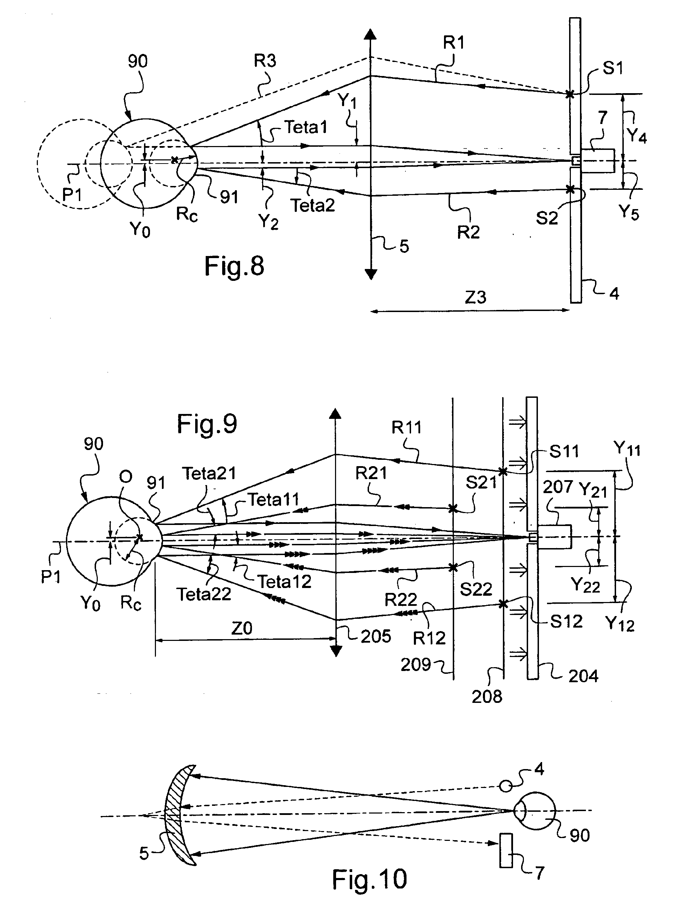

En variante, il sera également possible, comme illustré sur la figure 10, de décaler la source lumineuse 4 et la caméra 7 de telle sorte qu'aucune plaque semi-réfléchissante ne soit nécessaire. Toutefois, ce décalage induira un écart du reflet cornéen par rapport à la pupille de l'individu. Cet écart pourra alors soit être corrigé optiquement, à l'aide d'un ou de deux prismes, soit être pris en compte par le calculateur 8 au cours de ses calculs.

Calculateur

Ici, le calculateur 8 est adapté à traiter les signaux en sortie de la caméra 7 pour, d'une part, calculer l'écart inter-pupillaire ou les demi-écarts pupillaires des deux yeux de l'individu, et d'autre part, calculer au moins une grandeur kératométrique pour chaque oeil de cet individu.

Pour mémoire, on entend par écart inter-pupillaire la distance qui sépare les deux pupilles de l'individu et par demi-écart pupillaire la distance qui sépare l'un des yeux de l'individu et l'arête de son nez.

La grandeur kératométrique pourra par exemple correspondre au rayon de courbure de la cornée si cette dernière est sensiblement sphérique (dans le 5 cas où l'individu ne présente pas de défaut d'astigmatisme), ou à un ensemble de rayons de courbure locaux pour différentes directions, ou encore à une surface représentative de la surface de la cornée.

Le calculateur 8 est ici formé par une carte électronique embarquée dans le boîtier 2, par exemple du type ADSP-BF531 de la société Analog Devices. Il est équipé d'un afficheur LCD 11 qui est positionné au travers d'une ouverture agencée dans le boîtier 2 pour assurer l'affichage des mesures effectuées.

Eventuellement, on pourra prévoir que le kératomètre automatique 1 comporte en outre un système d'occultation monoculaire, constitué de deux écrans à diaphragme pilotés par le calculateur et disposés respectivement devant les deux fenêtres de mesure 9. Par réglage de la tension les alimentant, de tels écrans peuvent passer de l'état transparent à l'état d'occultation et inversement, indépendamment l'un de l'autre.

Sur la figure 8, on a représenté schématiquement le système optique d'imagerie 5, la source lumineuse 4 et le capteur photoélectrique 7 du kératomètre 1, ainsi que l'un des yeux 90 de l'individu. Cet oeil 90 est ici représenté avec une cornée 91 de forme sphérique, bien que ce ne soit que rarement le cas dans la réalité. On a également représenté sur cette figure les trajets optiques de deux rayons lumineux R1, R2 qui sont issus de deux diodes électroluminescentes 42 distinctes de la source lumineuse 4 et qui forment sur la cornée deux reflets dont les images sont visibles de manière nette par la caméra 7.

Second mode de réalisation du kératomètre

Dans un second mode de réalisation de l'invention représenté sur la figure 7, le kératomètre automatique 101 est adapté à réaliser simultanément ou tour à tour des mesures sur les deux yeux du porteur, au moyen d'un système optique d'imagerie apte à générer deux images sur chacune desquelles apparaît le reflet cornéen de l'un des deux yeux de l'individu.

Le système optique d'imagerie est plus précisément adapté à générer simultanément ou tour à tour deux images distinctes des deux reflets cornéens. On dit alors du kératomètre automatique 101 qu'il comporte deux voies optiques séparées.

Le système optique d'imagerie comporte pour cela deux lentilles de collimation 105A, 105B identiques chacune situées en regard de l'une des fenêtres de mesure 109 du boîtier 102. Elles sont positionnées dans ce boîtier 102 de manière que leurs axes optiques s'étendent parallèlement à l'axe longitudinal Z et que leurs plans focal image se confondent et se situent à faible distance de la fenêtre d'observation 110, du côté intérieur du boîtier 102. Une première lentille de collimation 105A forme une première voie et est adaptée à générer une image du reflet cornéen d'un premier oeil de l'individu tandis qu'une seconde lentille de collimation 105B forme une seconde voie et est adaptée à générer une image du reflet cornéen du second oeil de l'individu.

Dans ce mode de réalisation du kératomètre 101, il est prévu deux sources lumineuses 104A, 104B positionnées au voisinage du plan focal image commun des lentilles de collimation 105A, 105B. Une première source lumineuse 104A éclaire exclusivement le premier oeil de l'individu tandis qu'une seconde source lumineuse 104B éclaire exclusivement le second oeil de l'individu.

Telles que représentées sur la figure 7, les sources lumineuses sont chacune formées par une matrice de diodes électroluminescentes. Elles pourraient bien sûr être formées autrement, par exemple par une lampe superposée à un masque.

Le kératomètre 101 comporte par ailleurs ici deux capteurs photoélectriques constitués par deux caméras numériques 107A, 107B chacune agencées pour capturer l'image de l'un des reflets cornéens et prévues pour transformer cette image en un signal électrique représentatif. Une première caméra 107A est plus précisément agencée pour capter l'image générée par la première lentille de collimation 105A tandis que la seconde caméra 107B est agencée pour capter l'image générée par la seconde lentille de collimation 105B.

Ces caméras 107A, 107B sont préférentiellement positionnées au voisinage du plan focal image commun aux deux lentilles de collimation 105A, 105B. Elles pourront à cet effet être disposées soit au travers des plaques-support des matrices de diodes 104A, 104B, suivant deux axes parallèles à l'axe longitudinal Z du boîtier 102, soit perpendiculairement à cet axe, au moyen d'une ou de deux plaque(s) semi-réfléchissante(s) prévue(s) pour dévier les axes optiques des deux lentilles de collimation 105A, 105B.

Enfin, le calculateur est adapté à traiter les signaux en sortie des deux caméras 107A, 107B pour, d'une part, calculer l'écart inter-pupillaire ou les demi-écarts pupillaires des deux yeux de l'individu, et d'autre part, calculer au moins une grandeur kératométrique pour chaque oeil de cet individu.

Troisième mode de réalisation du kératomètre

Dans un troisième mode de réalisation de l'invention représenté schématiquement sur la figure 9, le kératomètre automatique est adapté à réaliser simultanément des mesures sur les deux yeux du porteur, au moyen d'un unique capteur photoélectrique 207 et d'une unique image plane sur laquelle apparaissent les reflets cornéens des deux yeux de l'individu.

Il comporte ici une unique voie optique, et présente une architecture homologue à celle du kératomètre automatique 1 représenté sur la figure 1. Il se distingue toutefois de ce dernier en ce que sa source lumineuse 204 et son capteur photoélectrique 207 ne sont pas nécessairement placés au voisinage du plan focal image de sa lentille de collimation 205.

Plus précisément ici, le système optique d'imagerie comporte une unique lentille de collimation 205 qui est adaptée à générer une image unitaire sur laquelle apparaissent l'un et l'autre des reflets cornéens des deux yeux de l'individu.

La source lumineuse 204 est quant à elle formée par une lampe superposée à deux masques 208, 209 distincts qui sont situés sur l'axe optique de la lentille de collimation 205, perpendiculairement à celui-ci, à des distances différentes du plan focal image de cette lentille de collimation 205.

La lampe comporte ici une pluralité de diodes électroluminescentes blanches positionnées derrière un diffuseur de lumière qui permet d'homogénéiser l'intensité lumineuse émise par ces diodes.

Chaque masque 208, 209 est ici formé par une plaque transparente pourvue d'une matrice de points opaques aptes à se réfléchir sur les cornées de l'individu. En variante, on pourrait également prévoir que chaque masque soit formé par un écran à cristaux liquides (LCD) transparent piloté par le calculateur pour afficher des motifs, comme par exemple une trame de points noirs sur fond transparent. Encore en variante, on pourrait utiliser des sources lumineuses sur deux plans distincts.

Le capteur photoélectrique est ici constitué d'une caméra numérique 207 qui est agencée pour capturer l'image des deux reflets cornéens formés par le système optique d'imagerie et qui est prévue pour transformer cette image en un signal électrique représentatif.

Cette caméra 207 est soit positionnée sur l'axe optique de la lentille de collimation 205, soit positionnée suivant un axe perpendiculaire à cet axe optique, auquel cas le kératomètre est équipé d'une plaque semi-réfléchissante adaptée à dévier à angle droit cet axe optique.

Enfin, le calculateur est adapté à traiter les signaux en sortie de la caméra pour, d'une part, calculer l'écart inter-pupillaire ou les demi-écarts pupillaires des deux yeux de l'individu, et d'autre part, calculer au moins une grandeur kératométrique pour chaque oeil de cet individu.

Sur la figure 9, on a représenté les trajets optiques de quatre rayons lumineux R11, R12, R21, R22, dont deux premiers rayons lumineux R11, R12 issus de deux points opaques S11, S12 d'un premier des deux masques 208 et deux seconds rayons lumineux R21, R22 issus de deux points opaques S21, S22 d'un second des deux masques 209. Ces quatre rayons lumineux R11, R12, R21, R22 forment ainsi sur chaque cornée quatre reflets dont les images sont visibles par la caméra 207.

Dans un autre mode de réalisation de l'invention, on peut prévoir d'utiliser, en lieu et place de la pluralité de sources lumineuses, une unique source lumineuse associée à une optique diffractive située entre cette unique source lumineuse et la lentille de collimation. Une telle optique diffractive permet en effet, à partir de l'unique source lumineuse, de générer une pluralité de faisceaux lumineux parallèles, orientés dans des directions différentes.

Il est en particulier possible d'utiliser une optique diffractive constituée de motifs se répétant de manière périodique suivant les axes horizontaux et verticaux. Ces motifs se distinguent du fait qu'il présentent une transmission lumineuse différente du reste de l'optique diffractive, ou un indice différent, ou encore, une épaisseur différente.

Ce mode de réalisation présente l'avantage de permettre une plus grande compacité du kératomètre, l'optique diffractive étant de taille similaire à celle de la lentille de collimation et d'épaisseur faible.

Dans ce mode de réalisation de l'invention, on peut prévoir que l'optique diffractive puisse être activée ou désactivée à volonté. Pour cela, cette optique pourra être montée de manière escamotable, ou pourra être activée électriquement (écran LCD ou ferroélectrique), ou pourra encore n'être adaptée à produire son effet que pour une longueur d'onde non visible de l'utilisateur, typiquement l'infrarouge.

<u>Procédé de kératométrie</u>

Le procédé de kératométrie selon l'invention sera ici mis en oeuvre par le kératomètre 1 représenté sur les figures 1 à 4.

L'observateur responsable de la mesure, qui est généralement un opticien, prend en main le boîtier 2 du kératomètre 1 et le démarre en appuyant sur un bouton marche/arrêt 12 (figure 1). Lors de cette mise en marche, le calculateur 8 commande automatiquement l'illumination de quatre des diodes électroluminescentes 42 de la source lumineuse 4, celles situées au centre de la plaque-support 41.

Puis, l'opticien positionne le boîtier 2 du kératomètre 1 sur le visage de l'individu et vérifie que celui-ci regarde bien dans la bonne direction, à savoir vers la source lumineuse 4. Pour cela, il regarde au travers de la fenêtre d'observation 10 et observe si les reflets cornéens sont bien centrés par rapport aux pupilles des yeux de l'individu. Il vérifie par ailleurs que l'individu est dans une position de regard stable, à savoir qu'il a bien les yeux immobiles et grand ouverts. Quand ces conditions sont réunies, il lance la mesure par appui sur un bouton de démarrage 13.

Fonctionnellement, comme le montre la figure 8, la source lumineuse 4 génère un reflet cornéen sur les deux yeux de l'individu, et ces reflets cornéens sont transmis par réflexion vers la caméra 7 grâce à la plaque semi-réfléchissante 6. L'image acquise par la caméra 7 est ensuite transmise au calculateur 8 qui repère la position du reflet de chaque diode électroluminescente sur la cornée en déterminant les coordonnées du barycentre des points adjacents d'intensité supérieure à un seuil. Le calculateur 8 peut ainsi déduire de la position de ces reflets les grandeurs kératométriques recherchées, ainsi que les écarts pupillaires ou l'écart inter-pupillaire de l'individu. Ces calculs nécessitent très peu de puissance et peuvent être réalisés simultanément à la capture d'image par la caméra 7.

En variante, comme le montre la figure 5, on pourrait prévoir que le kératomètre 1 soit équipé d'un socle 15 et que la mise en place du boîtier 2 sur le socle 15 déclenche l'envoi automatique des mesures à un logiciel de traitement d'un ordinateur 16 qui se chargerait du calcul des grandeurs kératométriques recherchées, ainsi que des écarts pupillaires ou de l'écart inter-pupillaire de l'individu. Ce logiciel de traitement pourra en particulier être hébergé par l'ordinateur situé dans le local où la mesure est effectuée, ou par un ordinateur situé à distance de ce local, par exemple chez un fabriquant de lentilles de contact.

Quoi qu'il en soit, le procédé de kératométrie automatique comporte selon l'invention plusieurs étapes successivement mises en oeuvre.

Au cours d'une première étape, le calculateur 8 commande l'illumination de l'ensemble des diodes électroluminescentes 42 de la source de lumière 4, ce qui a pour effet de simultanément éclairer les deux yeux de l'individu.

Au cours d'une seconde étape, le calculateur 8 commande à la caméra 7 de capturer un cliché sur lequel apparaît l'image des reflets cornéens de la source lumineuse 4 sur les deux yeux de l'individu. La caméra 7 convertit alors cette image en un signal numérique représentatif qu'elle transmet au calculateur 8.

Enfin, au cours d'une troisième étape, le calculateur 8 procède au calcul des grandeurs kératométriques des yeux de l'individu ainsi qu'au calcul de l'écart pupillaire ou des demi-écarts pupillaires des deux yeux de l'individu, comme cela sera décrit plus en détail dans la suite de cet exposé.

Le procédé de fonctionnement du kératomètre schématiquement représenté sur la figure 9 est identique à celui précédemment exposé. Seul le mode de calcul des grandeurs kératométriques diffère.

Quant au procédé de fonctionnement du kératomètre 100 représenté sur la figure 7, il diffère du procédé précédemment exposé en ce que les reflets cornéens des deux yeux peuvent être capturés simultanément ou tour à tour, pour autant que l'opticien s'assure que l'individu ne bouge pas entre les mesures.

Premier mode de calcul du rayon de courbure

Les grandeurs kératométriques ici acquises par le kératomètre 1 sont formées par les rayons de courbure Rc de la cornée en une pluralité de zones distinctes. Plus précisément ici, le calculateur 8 détermine les rayons de courbure Rc de chaque cornée en une pluralité d'arcs dont les extrémités sont formées par les reflets de deux diodes électroluminescentes adjacentes.

En référence à la figure 8, on peut illustrer ce calcul en détaillant l'algorithme de calcul du rayon de courbure de la cornée de l'oeil 90 de l'individu, entre les reflets des deux diodes S1, S2 représentées. Sur cette figure 8, le plan moyen P1 correspond au plan horizontal qui comprend l'axe optique de la lentille de collimation 5.

Pour ce calcul, l'oeil 90 de l'individu est considéré sphérique entre les deux reflets. Le centre de cette sphère peut être légèrement décalé par rapport au plan moyen P1, du fait d'une erreur de positionnement du boîtier 2 contre le visage de l'individu. Ce décalage en hauteur par rapport au plan moyen P1 est référencé Y0.

Lorsqu'elle est allumée, la première diode électroluminescente S1 diffuse un faisceau de lumière dont seul un rayon lumineux R1 présente une partie incidente qui passe au travers de la lentille de collimation 5 pour impacter la cornée de l'oeil 90 et une partie réfléchie qui suit un axe parallèle au plan moyen P1 puis qui traverse la lentille de collimation 5 pour former une image nette et visible par la caméra 7.

De la même manière, seul un rayon lumineux R2 issu de la seconde diode électroluminescente S2 présente une partie incidente qui passe au travers de la lentille de collimation 5 pour impacter la cornée de l'oeil 90 et une partie réfléchie qui suit un axe parallèle au plan moyen P1 puis qui traverse la lentille de collimation 5 pour former une image nette et visible par la caméra 7.

Les parties incidente et réfléchie de chaque rayon lumineux R1, R2 forment un angle Teta1, Teta2 fonction de l'architecture du kératomètre 1. Plus précisément, les valeurs de ces angles Teta1 et Teta2 dépendent des positions des deux diodes électroluminescentes S1, S2 considérées par rapport au plan moyen P1 et peuvent être déterminées au moyen des calculs suivant :

$$\text{Teta1} = \text{Arctan}\ (Y4/Z3),$$

$$\text{Teta2} = \text{Arctan}\ (Y5/Z3),$$

où Y4 correspond à la hauteur de la diode S1 par rapport au plan moyen P1, où Y5 correspond à la hauteur de la diode S2 par rapport au plan moyen P1 et où Z3 correspond à la distance focale de la lentille de collimation 5. Ces valeurs d'angle sont prédéterminées et mémorisées par le calculateur 8.

Pour déterminer la valeur du rayon de courbure Rc considéré, le calculateur 8 détermine sur l'image acquise par la caméra 7 les hauteurs Y1, Y2 par rapport au plan moyen P1 des reflets des deux rayons lumineux R1, R2.

Puis, le calculateur résout le système suivant de deux équations à deux inconnues :

$$\text{Arcsin}((Y1-Y0)/Rc) = \text{Teta1} / 2$$

$$\text{Arcsin}((Y2-Y0)/Rc) = \text{Teta2} / 2,$$

où la hauteur Y0 et le rayon de courbure Rc sont les deux inconnues.

De cette manière, le calculateur 8 obtient la valeur du rayon de courbure Rc de la cornée entre les reflets des deux rayons lumineux R1, R2 considérés.

Le calculateur peut également directement calculer la valeur du rayon de courbure Rc au moyen de la formule suivante :

$$Rc = (Y1 - Y2) / [\sin\ (\text{Teta1} / 2) - \sin\ (\text{Teta2} / 2)].$$

Puis, le calculateur 8 réitère ce calcul sur l'ensemble des arcs considérés, de manière à obtenir un maillage précis des rayons de courbure des deux cornées de l'individu.

L'invention n'est pas limitée à ce mode de calcul, et il existe d'autres méthodes connues pour obtenir un maillage précis des rayons de courbure telle que celle définie dans l'ouvrage de Sicam et al., « J. Opt. Soc. Am. A/Vol. 21, No. 7/July 2004 », et plus précisément celle exposée dans le chapitre « Corneal surface reconstruction algorithm that uses Zernike polynomial representation », pages 1300 à 1306.

Enfin, le calculateur 8 calcule le rayon de courbure moyen de chaque cornée et l'affiche sur l'afficheur LCD 11. On peut également prévoir qu'il transmette l'ensemble du maillage de rayons de courbure à l'ordinateur 16 lorsque l'opticien repose le kératomètre sur son socle 15.

L'architecture du kératomètre 1 illustré sur la figure 8 est ainsi particulièrement avantageuse dans la mesure où le calcul des rayons de courbure Rc ne dépend pas de la distance séparant l'oeil de la lentille de collimation 5. En effet, comme le montre le trajet du rayon lumineux R3 représenté en pointillés sur la figure 8, puisque la caméra 7 et la source lumineuse 4 sont toutes deux situées dans le plan focal image de la lentille de collimation 5, la distance séparant l'oeil et la lentille de collimation n'a aucune influence sur les hauteurs Y1, Y2 mesurées. Par conséquent, grâce à cette architecture, les erreurs de positionnement de l'individu par rapport au boîtier 2 n'ont aucune influence sur la précision des calculs des rayons de courbure Rc.

Second mode de calcul du rayon de courbure

Lorsque le kératomètre est du type de celui représenté schématiquement sur la figure 9, le calculateur met en oeuvre un algorithme de calcul du rayon de courbure Rc qui est légèrement différent de celui présenté précédemment. En effet, la caméra 207 et/ou les masques 208, 209 n'étant pas positionnés au plan focal image de la lentille de collimation 205, la distance Z0 séparant l'oeil 90 et cette lentille de collimation 205 présente une influence sur le calcul du rayon de courbure Rc.

Pour ce calcul, l'oeil 90 de l'individu est considéré sphérique sur l'arc qui passe par les reflets des quatre points opaques S11, S12, S21, S22 sur la rétine.

Les quatre rayons lumineux R11, R12, R21, R22 présentent une partie incidente et une partie réfléchie par la cornée inclinées l'une par rapport à l'autre d'un angle respectivement noté Teta11, Teta12, Teta21, Teta22.

Les valeurs de ces angles Teta11, Teta12, Teta21, Teta22 sont fonction, d'une part, de la distance Z0 séparant l'oeil 90 et la lentille de collimation 205, et, d'autre part, des hauteurs Y11, Y12, Y21, Y22 par rapport au plan moyen P1 des reflets des quatre rayons lumineux R11, R12, R21, R22.

La distance Z0 n'étant initialement pas connue du calculateur, ce dernier est programmé pour faire une hypothèse sur la valeur de cette distance Z0. La fonction mathématique donnant les valeurs de ces angles Teta11, Teta12, Teta21, Teta22 est directement déterminée en fonction des paramètres de construction du kératomètre.

Plus précisément, étant donnée la forme du boîtier et la variété de formes de visages des individus, on sait que la distance Z0 sera comprise dans un intervalle déterminé, allant ici de 150 à 250 millimètres.

Par conséquent, le calculateur est alors programmé pour calculer les valeurs que présenteraient les quatre angles Teta11, Teta12, Teta21, Teta22 pour différentes valeurs de distances Z0. Il est ici plus précisément programmé pour faire varier la distance Z0 de 150 à 250 millimètres, avec un pas prédéterminé ici égal à 1 millimètre, et pour calculer pour chacune de ces valeurs de distance Z0 les valeurs que présenteraient les quatre angles Teta11, Teta12, Teta21, Teta22.

Le calculateur obtient ainsi une centaine de triplets (Teta11i, Teta12i, Z0i) associés aux points opaques S11, S12 du premier masque 208 et une centaine de triplets (Teta21 j, Teta22j, Z0j) associés aux points opaques S21, S22 du second masque 209. On comprend ici que parmi chacun de ces triplets (Teta11i, Teta12i, Z0i), seul un correspond à la réalité. Il s'agit alors de déterminer lequel.

Pour cela, le calculateur 8 détermine sur l'image acquise par la caméra 207 les hauteurs Y11, Y12, Y21, Y22 par rapport au plan moyen P1 des reflets des quatre rayons lumineux R11, R12, R21, R22.

Puis, le calculateur résout, pour chaque triplet, le système suivant de deux équations à deux inconnues :

$$\text{Arcsin}((Y11i-Y0)/Rci) = Teta11i(Z0i, Y11i) / 2$$

$$\text{Arcsin}((Y12i-Y0)/Rci) = Teta12i(Z0i, Y12i) / 2,$$

avec i allant de 1 à 100,
d'où

$$Rci = (Y11i - Y12i) / [\sin(Teta11i / 2) - \sin(Teta12i / 2)]$$

et

$$\text{Arcsin}((Y21j - Y0) / Rcj) = Teta21j(Z0j, Y21j) / 2$$

$$\text{Arcsin}((Y22j - Y0) / Rcj) = Teta22j(Z0j, Y22j) / 2,$$

avec j allant de 1 à 100,
d'où

$$Rcj = (Y21j - Y22j) / [\sin(Teta21j / 2) - \sin(Teta22j / 2)].$$

De cette manière, le calculateur 8 obtient deux ensembles de doublets (Rci, Z0i) et (Rcj, Z0j).

Enfin, le calculateur recherche un doublet commun aux deux ensembles, c'est-à-dire un doublet du premier ensemble dont les valeurs de distance Z0 et de rayon de courbure Rc sont égales aux valeurs correspondantes d'un doublet du second ensemble.

Puisque les valeurs de distance Z0 et de rayon de courbure Rc sont uniques, le calculateur obtient ainsi la valeur réelle du rayon de courbure Rc de l'arc considéré sur la cornée.

Le calculateur 8 peut ensuite réitérer ce calcul sur l'ensemble des arcs considérés, de manière à obtenir un maillage précis des rayons de courbure Rc des deux cornées de l'individu.

L'architecture du kératomètre 1 illustré sur la figure 9 est ici avantageuse dans la mesure où elle permet d'acquérir la valeur de la distance Z0.

Cette valeur de distance Z0 permet en effet de s'assurer que lors de la mesure, les yeux du porteur étaient placés à une distance du boîtier telle que la caméra 207 pouvait obtenir une image nette de ses cornées.

Cette valeur de distance Z0 permet en outre avantageusement de déterminer les demi-écarts pupillaires du porteur en vision de près, comme cela sera décrit plus en détail dans la suite de cet exposé.

Mesure des écarts pupillaires

Pour mesurer l'écart inter-pupillaire de l'individu, le calculateur 8 détermine sur l'image acquise par la caméra 7 les 36 distances séparant les reflets de chaque diode électroluminescente sur les deux yeux de l'individu. Puis, il calcule une distance moyenne qui correspond à la distance, sur l'image acquise, entre les deux pupilles des yeux de l'individu. Connaissant le grossissement du système optique d'imagerie 5, le calculateur 8 peut ainsi en déduire la valeur de l'écart inter-pupillaire de l'individu et commander l'affichage de cet écart sur l'afficheur LCD 11.

Cette mesure peut être complétée ou remplacée par une mesure des demi-écarts pupillaires en monoculaire, au moyen des deux écrans à diaphragme commandés électriquement.

L'un des écrans pouvant être réglé à l'état d'occultation pour n'assurer une vue que de l'autre oeil, on peut aussi détecter des problèmes de strabisme en exploitant le fait que, les deux yeux ne pouvant focaliser ensemble sur la source lumineuse 4, l'un des yeux est amené à bouger à chaque fois que les deux écrans changent d'état.

On peut aussi détecter des problèmes d'amblyopie (cas d'un oeil qui ne voit pas ou peu) puisque, selon que l'oeil qui voit est occulté ou non, l'oeil amblyope donne un demi-écart pupillaire différent.

Enfin, le calculateur 8 peut comporter un module de détermination de l'orientation de la ligne d'horizon à partir des signaux représentatifs des images des reflets cornéens des deux yeux de l'individu. Ce module déterminera ainsi l'orientation, sur l'image acquise par la caméra 7, de la droite passant par les centres des deux pupilles de l'individu.

Ceci permet ainsi de donner l'orientation suivant cette ligne d'horizon de la méridienne de la cornée présentant le rayon le plus important ou le plus faible en cas d'astigmatisme et par conséquent de donner l'angle d'astigmatisme par rapport à cette ligne d'horizon.

**Revendications**

**1.** Procédé de kératométrie automatique pour la mesure d'au moins une grandeur kératométrique (Rc) relative à la géométrie de la cornée (91) de chacun des deux yeux (90) d'un individu, comportant les étapes de :

- éclairer chaque oeil (90) au moyen d'au moins une source lumineuse (4 ; 104A, 104B) de configuration géométrique connue,
- capturer une image du reflet cornéen de ladite source lumineuse (4 ; 104A, 104B) sur chaque oeil (90) de l'individu, tour à tour ou simultanément, au moyen d'un capteur photoélectrique (7 ; 107A, 107B) couplé à un système optique d'imagerie (5 ; 105A, 105B) et adapté à convertir cette image en un signal qui en est représentatif, sans déplacement relatif du visage de l'individu par rapport au capteur photoélectrique (7 ; 107A, 107B),
- déduire de ce signal ladite grandeur kératométrique (Rc) de l'oeil (90), **caractérisé en ce qu'**il comporte une étape de calcul, à partir des signaux représentatifs des images des reflets cornéens de ladite source lumineuse (4 ; 104A, 104B) sur les deux yeux (90) et du grossissement du système optique d'imagerie (5 ; 105A, 105B), de l'écart inter-pupillaire ou des demi-écarts pupillaires des deux yeux (90) de l'individu.

**2.** Procédé selon la revendication 1, dans lequel les deux yeux (90) sont éclairés simultanément, dans lequel les images des reflets cornéens des deux yeux (90) sont capturées simultanément et dans lequel la déduction de ladite grandeur kératométrique (Rc) de chaque oeil est réalisée au moins à partir dudit signal relatif au reflet cornéen de cet oeil (90) capturé simultanément au reflet de l'autre oeil (90).

**3.** Procédé selon l'une des revendications précédentes, dans lequel à la fois l'un et l'autre des deux yeux (90) sont éclairés au moyen d'une source lumineuse commune (4).

**4.** Procédé selon l'une des revendications 1 et 2, dans lequel les deux yeux (90) sont éclairés par deux sources lumineuses (104A, 104B), dont une première source lumineuse (1 04A) éclairant exclusivement l'un des deux yeux (90) et une seconde source lumineuse (104B) éclairant exclusivement l'autre oeil (90).

**5.** Procédé selon l'une des revendications précédentes, comportant de plus l'étape d'acquérir, à partir des signaux représentatifs des images des reflets cornéens des deux yeux (90), la ligne d'horizon.

**6.** Procédé selon l'une des revendications précédentes, dans lequel, lors de la capture d'image, le boîtier (2, 102) qui renferme ladite source lumineuse (4 ; 104A, 104B) et ledit capteur photoélectrique (7 ; 107A, 107B) est porté à la main par un opérateur distinct dudit individu.

**7.** Kératomètre automatique (1 ; 101) adapté à la mesure d'au moins une grandeur kératométrique (Rc) relative à la géométrie de la cornée (91) de chacun des deux yeux (90) d'un individu, comportant :

- un boîtier (2 ; 102),
- au moins une source lumineuse (4 ; 104A, 104B) de configuration géométrique connue montée sur le boîtier (2 ; 102) de manière à éclairer l'oeil (90) à mesurer de l'individu,
- un système optique d'imagerie (5 ; 105A, 105B) de grossissement connu, comportant une ou deux lentilles, monté sur le boîtier (2 ; 102) et agencé pour fournir au moins une image plane du reflet cornéen de ladite source lumineuse (4 ; 104A, 104B) sur chaque oeil (90) de l'individu,
- au moins un capteur photoélectrique (7 ; 107A, 107B) monté sur le boîtier (2 ; 102) pour capturer, tour à tour ou simultanément, les images des reflets cornéens des deux yeux (90) de l'individu, sans déplacement relatif du visage de l'individu par rapport au capteur photoélectrique (7 ; 107A, 107B), et adapté à convertir cette image en un signal qui en est représentatif,
- un calculateur (8) adapté à traiter ledit signal pour calculer ladite grandeur kératométrique (Rc) de l'oeil (90), **caractérisé en ce que** le calculateur (8) est adapté à traiter les signaux représentatifs des images des reflets cornéens des deux yeux, pour calculer l'écart inter-pupillaire ou les demi-écarts pupillaires des deux yeux (90) de l'individu en fonction du grossissement du système optique d'imagerie (5 ; 105A, 105B).

**8.** Kératomètre automatique (1) selon la revendication précédente, dans lequel ladite au moins une source lumineuse (4) est agencée pour éclairer les deux yeux (90) simultanément, dans lequel ledit système optique d'imagerie (5) est agencé pour fournir simultanément des images des deux reflets cornéens de ladite source lumineuse (4) sur les deux yeux (90), dans lequel ledit capteur photoélectrique (7) est agencé pour capturer simultanément ces images et dans lequel le calculateur (8) est adapté à traiter lesdits signaux représentatifs de ces images pour calculer les grandeurs kératométriques (Rc) des deux yeux (90).

**9.** Kératomètre automatique (1) selon la revendication précédente, dans lequel ledit système optique d'imagerie (5) est adapté à générer une image unitaire englobant les reflets cornéens des deux yeux (90).

**10.** Kératomètre automatique (1) selon la revendication précédente, dans lequel ledit système optique d'imagerie comporte au moins l'un des éléments suivants :

- une lentille (5) ayant au moins un diamètre horizontal d'au moins dix centimètres et dont l'axe optique (Z), éventuellement renvoyé ou dévié, aboutit au capteur photoélectrique (7),
- deux parties de lentilles ou deux doublets équivalant à une lentille unique dont l'axe optique, éventuellement renvoyé ou dévié, aboutit au capteur photoélectrique.

**11.** Kératomètre automatique (101) selon la revendication 7, dans lequel ledit système optique d'imagerie comporte deux voies séparées, avec une première voie (105A) adaptée à générer une image du reflet cornéen de l'un des deux yeux (90) et une seconde voie (105B) adaptée à générer une image du reflet cornée de l'autre oeil (90), dans lequel ledit au moins un capteur photoélectrique comporte un premier capteur photoélectrique (107A) agencé pour capter l'image générée par la première voie et un second capteur photoélectrique (107B) agencé pour capter l'image générée par la seconde voie.

**12.** Kératomètre automatique (1) selon l'une des revendications 7 à 11, dans lequel ladite au moins une source lumineuse comporte au moins une source lumineuse commune (4) éclairant à la fois l'un et l'autre des deux yeux (90).

**13.** Kératomètre automatique (101) selon l'une des revendications 7 à 11, dans lequel ladite au moins une source comporte au moins une première source lumineuse (104A) éclairant exclusivement l'un des deux yeux (90) et une seconde source lumineuse (104B) éclairant exclusivement l'autre oeil (90).

**14.** Kératomètre automatique (1 ; 101) selon l'une des revendications 7 à 13, dans lequel la source lumineuse (4 ; 104) est disposée au plan focal image du système optique d'imagerie (5 ; 105).

**15.** Kératomètre automatique (1 ; 101) selon l'une des revendications 7 à 14, dans lequel le capteur photoélectrique (7 ; 107) est disposé au plan focal image du système optique d'imagerie (5 ; 105).

**Patentansprüche**

**1.** Automatisches Keratometrieverfahren zur Messung mindestens einer keratometrischen Größe (Rc) bezüglich der Geometrie der Hornhaut (91) jedes der zwei Augen (90) einer Person, das die folgenden Schritte aufweist:

- Beleuchten jedes Auges (90) mittels mindestens einer Lichtquelle (4; 104A, 104B) bekannter geometrischer Konfiguration,
- Auffangen eines Bilds des Hornhautreflexes der Lichtquelle (4; 104A, 104B) auf jedem Auge (90) der Person, nacheinander oder gleichzeitig, mittels eines fotoelektrischen Sensors (7; 107A, 107B), der mit einem optischen Abbildungssystem (5; 105A, 105B) gekoppelt und geeignet ist, um dieses Bild in ein Signal umzuwandeln, das dafür repräsentativ ist, ohne relative Verschiebung des Gesichts der Person bezüglich des fotoelektrischen Sensors (7; 107A, 107B),
- Ableiten der keratometrischen Größe (Rc) des Auges (90) von diesem Signal,
**dadurch gekennzeichnet, dass** es einen Schritt der Berechnung, ausgehend von den für die Bilder der Hornhautreflexe der Lichtquelle (4; 104A, 104B) auf den zwei Augen (90) repräsentativen Signale und der Vergrößerung des optischen Abbildungssystems (5; 105A, 105B), des Zwischenpupillenabstands oder der Halbzwischenpupillenabstände der zwei Augen (90) der Person aufweist.

**2.** Verfahren nach Anspruch 1, wobei die zwei Augen (90) gleichzeitig beleuchtet werden, wobei die Bilder der Hornhautreflexe der zwei Augen (90) gleichzeitig aufgefangen werden, und wobei die Ableitung der keratometrischen Größe (Rc) jedes Auges mindestens ausgehend von dem Signal bezüglich des Hornhautreflexes dieses Auges (90) durchgeführt wird, das gleichzeitig mit dem Reflex des anderen Auges (90) aufgefangen wird.

**3.** Verfahren nach einem der vorhergehenden Ansprüche, wobei sowohl das eine als auch das andere der zwei Augen (90) mittels einer gemeinsamen Lichtquelle (4) beleuchtet wird.

**4.** Verfahren nach einem der Ansprüche 1 und 2, wobei die zwei Augen (90) von zwei Lichtquellen (104A, 104B) beleuchtet werden, von denen eine erste Lichtquelle (104A) ausschließlich eines der zwei Augen (90) und eine zweite Lichtquelle (104B) ausschließlich das andere Auge (90) beleuchtet.

**5.** Verfahren nach einem der vorhergehenden Ansprüche, das außerdem den Schritt aufweist, ausgehend von den für die Bilder der Hornhautreflexe der zwei Augen (90) repräsentativen Signalen, die Horizontlinie zu erlangen.

**6.** Verfahren nach einem der vorhergehenden Ansprüche, wobei beim Auffangen des Bilds das Gehäuse (2, 102), das die Lichtquelle (4; 104A, 104B) und den fotoelektrischen Sensor (7; 107A, 107B) enthält, von einem Bediener in der Hand gehalten wird, der sich von der Person unterscheidet.

**7.** Automatischer Keratometer (1; 101), der für die Messung mindestens einer keratometrischen Größe (Rc) bezüglich der Geometrie der Hornhaut (91) jedes der zwei Augen (90) einer Person geeignet ist, der aufweist:

- ein Gehäuse (2; 102),
- mindestens eine Lichtquelle (4; 104A, 104B) bekannter geometrischer Konfiguration, die so auf das Gehäuse (2; 102) montiert ist, dass sie das zu messende Auge (90) der Person beleuchtet,
- ein optisches Abbildungssystem (5; 105A, 105B) bekannter Vergrößerung, das eine oder zwei Linsen aufweist, auf das Gehäuse (2; 102) montiert und eingerichtet ist, um mindestens ein ebenes Bild des Hornhautreflexes der Lichtquelle (4; 104A, 104B) auf jedem Auge (90) der Person zu liefern,
- mindestens einen auf das Gehäuse (2; 102) montierten fotoelektrischen Sensor (7; 107A, 107B), um nacheinander oder gleichzeitig die Bilder der Hornhautreflexe der zwei Augen (90) der Person ohne relative Verschiebung des Gesichts der Person bezüglich des fotoelektrischen Sensors (7; 107A, 107B) aufzufangen, und geeignet, um dieses Bild in ein Signal umzuwandeln, das dafür repräsentativ ist,
- einen Rechner (8), der geeignet ist, um das Signal zu verarbeiten, um die keratometrische Größe (Rc) des Auges (90) zu berechnen,
**dadurch gekennzeichnet, dass** der Rechner (8) geeignet ist, um die für die Bilder der Hornhautreflexe der zwei Augen repräsentativen Signale zu verarbeiten, um den Zwischenpupillenabstand oder die Halbzwischenpupillenabstände der zwei Augen (90) der Person abhängig von der Vergrößerung des optischen Abbildungssystems (5; 105A, 105B) zu berechnen.

8. Automatischer Keratometer (1) nach dem vorhergehenden Anspruch, wobei die mindestens eine Lichtquelle (4) eingerichtet ist, um die zwei Augen (90) gleichzeitig zu beleuchten, wobei das optische Abbildungssystem (5) eingerichtet ist, um gleichzeitig Bilder der zwei Hornhautreflexe der Lichtquelle (4) auf den zwei Augen (90) zu liefern, wobei der fotoelektrische Sensor (7) eingerichtet ist, um gleichzeitig diese Bilder aufzufangen, und wobei der Rechner (8) geeignet ist, um die für diese Bilder repräsentativen Signale zu verarbeiten, um die keratometrischen Größen (Rc) der zwei Augen (90) zu berechnen.

9. Automatischer Keratometer (1) nach dem vorhergehenden Anspruch, wobei das optische Abbildungssystem (5) geeignet ist, um ein einheitliches Bild zu erzeugen, das die Hornhautreflexe der zwei Augen (90) umfasst.

10. Automatischer Keratometer (1) nach dem vorhergehenden Anspruch, wobei das optische Abbildungssystem mindestens eines der folgenden Elemente aufweist:

- eine Linse (5), die mindestens einen horizontalen Durchmesser von mindestens zehn Zentimetern hat und deren optische Achse (Z), ggf. rückgeführt oder abgelenkt, zum fotoelektrischen Sensor (7) führt,
- zwei Linsenbereiche oder zwei einer einzigen Linse entsprechende Dubletten, deren optische Achse, ggf. rückgeführt oder abgelenkt, zum fotoelektrischen Sensor führt.

11. Automatischer Keratometer (101) nach Anspruch 7, wobei das optische Abbildungssystem zwei getrennte Pfade aufweist, mit einem ersten Pfad (105A), der geeignet ist, um ein Bild des Hornhautreflexes eines der zwei Augen (90) zu erzeugen, und einem zweiten Pfad (105B), der geeignet ist, um ein Bild des Hornhautreflexes des anderen Auges (90) zu erzeugen, wobei der mindestens eine fotoelektrische Sensor einen ersten fotoelektrischen Sensor (107A), der eingerichtet ist, um das vom ersten Pfad erzeugte Bild aufzufangen, und einen zweiten fotoelektrischen Sensor (107B) aufweist, der eingerichtet ist, um das vom zweiten Pfad erzeugte Bild aufzufangen.

12. Automatischer Keratometer (1) nach einem der Ansprüche 7 bis 11, wobei die mindestens eine Lichtquelle mindestens eine gemeinsame Lichtquelle (4) aufweist, die gleichzeitig das eine und das andere der zwei Augen (90) beleuchtet.

13. Automatischer Keratometer (101) nach einem der Ansprüche 7 bis 11, wobei die mindestens eine Quelle mindestens eine erste Lichtquelle (104A), die ausschließlich eines der zwei Augen (90) beleuchtet, und eine zweite Lichtquelle (104B) aufweist, die ausschließlich das andere Auge (90) beleuchtet.

14. Automatischer Keratometer (1; 101) nach einem der Ansprüche 7 bis 13, wobei die Lichtquelle (4; 104) in der Bildbrennebene des optischen Abbildungssystems (5; 105) angeordnet ist.

15. Automatischer Keratometer (1; 101) nach einem der Ansprüche 7 bis 14, wobei der fotoelektrische Sensor (7; 107) in der Bildbrennebene des optischen Abbildungssystems (5; 105) angeordnet ist.

**Claims**

1. Automatic keratometry method for measuring at least one keratometric quantity (Rc) related to the geometry of the cornea (91) of each of the two eyes (90) of an individual, comprising steps of:

- illuminating each eye (90) by means of at least one light source (4; 104A, 104B) having a known geometric configuration;
- capturing an image of the corneal reflection of said light source (4; 104A, 104B) from each eye (90) of the individual, turn-by-turn or simultaneously, by means of a photoelectric sensor (7; 107A, 107B) that is coupled to an imaging optical system (5; 105A, 105B) and suitable for converting this image into a signal that is representative thereof, without the face of the individual moving relative to the photoelectric sensor (7; 107A, 107B); and
- deducing said keratometric quantity (Rc) of the eye (90) from said signal,
**characterised in that** it comprises a step of calculating, from signals representative of the images of corneal reflections of said light source (4; 104A, 104B) from the two eyes (90) and from the magnification of the imaging optical system (5; 105A, 105B), the interpupillary distance or half-pupillary distances of the two eyes (90) of the individual.

2. Method according to Claim 1, in which the two eyes (90) are illuminated simultaneously, in which the images of the corneal reflections from the two eyes (90) are captured simultaneously, and in which said keratometric quantity (Rc) of each eye is deduced at least from said signal related to the corneal reflection from this eye (90) captured simultaneously with the reflection from the other eye (90).

3. Method according to one of the preceding claims, in which both the two eyes (90) are illuminated by means of a common light source (4).

4. Method according to one of Claims 1 and 2, in which the two eyes (90) are illuminated by two light sources (104A, 104B), namely a first light source (104A) illuminating only one of the two eyes (90) and a second light source (104B) illuminating only the other eye (90).

5. Method according to one of the preceding claims, in addition comprising a step of acquiring, from signals representative of images of corneal reflections from the two eyes (90), the horizon line.

6. Method according to one of the preceding claims, in which, during the image capture, the housing (2, 102) that contains said light source (4; 104A, 104B) and said photoelectric sensor (7; 107A, 107B) is hand-held by an operator who is not said individual.

7. Automatic keratometer (1; 101) suitable for measuring at least one keratometric quantity (Rc) related to the geometry of the cornea (91) of each of the two eyes (90) of an individual, comprising:

 - a housing (2; 102);
 - at least one light source (4; 104A, 104B) having a known geometric configuration, which light source is mounted in the housing (2; 102) so as to illuminate the eye (90) to be measured of the individual;
 - an imaging optical system (5; 105A, 105B) of known magnification, comprising one or two lenses, which system is mounted in the housing (2; 102) and arranged to deliver at least one planar image of the corneal reflection of said light source (4; 104A, 104B) from each eye (90) of the individual;
 - at least one photoelectric sensor (7; 107A, 107B) mounted in the housing (2; 102) in order to capture, turn-by-turn or simultaneously, the images of corneal reflections from the two eyes (90) of the individual, without the face of the individual moving relative to the photoelectric sensor (7; 107A, 107B), and suitable for converting this image into a signal that is representative thereof; and
 - a processor (8) suitable for processing said signal in order to calculate said keratometric quantity (Rc) of the eye (90),
**characterised in that** the processor (8) is suitable for processing the signals representative of the images of corneal reflections from the two eyes, in order to calculate the interpupillary distance or half-pupillary distances of the two eyes (90) of the individual depending on the magnification of the imaging optical system (5; 105A, 105B).

8. Automatic keratometer (1) according to the preceding claim, in which said at least one light source (4) is arranged to illuminate both eyes (90) simultaneously, in which said imaging optical system (5) is arranged to deliver simultaneously images of two corneal reflections of said light source (4) from the two eyes (90), in which said photoelectric sensor (7) is arranged to capture simultaneously these images, and in which the processor (8) is suitable for processing said signals representative of these images in order to calculate the keratometric quantities (Rc) of the two eyes (90).

9. Automatic keratometer (1) according to the preceding claim, in which said imaging optical system (5) is suitable for generating a unitary image encompassing the corneal reflections of both eyes (90).

10. Automatic keratometer (1) according to the preceding claim, in which said imaging optical system comprises at least one of the following elements:

- a lens (5) at least one horizontal diameter of which is at least 10 cm, and the optical axis (Z) of which, optionally reflected or deviated, leads to the photoelectric sensor (7); and/or
- two lens portions or two doublets equivalent to a single lens the optical axis of which, optionally reflected or deviated, leads to the photoelectric sensor.

11. Automatic keratometer (101) according to Claim 7, in which said imaging optical system comprises two separate channels, with a first channel (105A) suitable for generating an image of the corneal reflection from one of the two eyes (90), and a second channel (105B) suitable for generating an image of the corneal reflection from the other eye (90), in which said at least one photoelectric sensor comprises a first photoelectric sensor (107A) arranged to sense the image generated in the first channel, and a second photoelectric sensor (107B) arranged to sense the image generated in the second channel.

12. Automatic keratometer (1) according to one of Claims 7 to 11, in which said at least one light source comprises at least one common light source (4) illuminating both of the two eyes (90).

13. Automatic keratometer (101) according to one of Claims 7 to 11, in which said at least one source comprises at least one first light source (104A) illuminating only one of the two eyes (90), and a second light source (104B) illuminating only the other eye (90).

14. Automatic keratometer (1; 101) according to one of Claims 7 to 13, in which the light source (4; 104) is placed in the image focal plane of the imaging optical system (5; 105).

15. Automatic keratometer (1; 101) according to one of Claims 7 to 14, in which the photoelectric sensor (7; 107) is placed in the image focal plane of the imaging optical system (5; 105).

Fig.1

Fig.2

Fig.3

Fig.4

Fig.5

Fig.6

Fig.7

Fig.8

Fig.9

Fig.10

**EP 2 168 474 B1**

## RÉFÉRENCES CITÉES DANS LA DESCRIPTION

**Documents brevets cités dans la description**

- US 4597648 A
- WO 0211612 A
- US 5777718 A

**Littérature non-brevet citée dans la description**

- Corneal surface reconstruction algorithm that uses Zernike polynomial representation. **SICAM et al.** J. Opt. Soc. Am. A. Juillet 2004, vol. 21, 1300-1306